# EUROPEAN PATENT APPLICATION

(11) **EP 4 458 384 A1**
(43) Date of publication of application: **06.11.2024**
(21) Application number: 22919044.2
(22) Date of filing: 14.12.2022
(51) Int. Cl.: A61M 5/142

(54) **DRUG SOLUTION INJECTION DEVICE**

(30) Priority: 04.01.2022 KR 20220001043; 28.04.2022 KR 20220052828
(71) Applicant: Eoflow Co., Ltd., Seongnam-si, Gyeonggi-do 13605 (KR)
(72) Inventor: KIM, Ki Ung, Incheon 21404 (KR); BANG, Won Kyung, Seoul 08239 (KR); KIM, Seung Ha, Goyang-si Gyeonggi-do 10558 (KR); HAN, Sang Hyun, Incheon 21549 (KR)
(74) Representative: AWA Sweden AB
(86) International application number: PCT/KR2022/020323
(87) International publication number: WO 2023/132507

(57) **Abstract**

A medical liquid injection device according to an embodiment of the present disclosure may include: a reservoir assembly storing medical liquid in a storage space defined by a reservoir and a plunger moving linearly inside the reservoir, a needle assembly connected fluidly to the reservoir assembly to discharge the stored medical liquid, and a driving unit transmitting a driving force generated by a driving module to the plunger, wherein the driving unit includes a rod member coupled to the plunger to move linearly together with the plunger, a wheel member rotating by the driving force, and a connection member having a catching portion that is movable in an axial direction of the wheel member and the rotation thereof with respect to the wheel member is limited around the axial direction.

## Description

### Technical Field

The present disclosure relates to a medical liquid injection device.

### Background Art

In general, a medical liquid injection device is used to inject medical liquid such as insulin into a patient. A medical liquid injection device is used by medical professionals such as doctors and nurses, but in most cases, it is used by the general public, such as the patients themselves or guardians.

Accordingly, a patch-type medical liquid injection device that is attached to a human body for a set period is being developed, and this medical liquid injection device can be used by attaching a patch to a human body, such as a patient's abdomen or waist, for a set period.

When a medical liquid injection device is attached to a patient's body and a needle and a cannula provided in the medical liquid injection device are inserted into the patient's body, the medical liquid stored inside the medical liquid injection device may be injected into the patient's body through the needle and the cannula.

To increase the effect through injecting medical liquid, a medical liquid injection device needs to be controlled to precisely inject medical liquid to a user.

When attached to a human body, a medical liquid injection device needs to be comfortable to wear, convenient to use, durable, and driven at low power. In particular, since a medical liquid injection device is used by attaching it directly to a patient's skin, it is important for a user to conveniently and safely drive the medical liquid injection device.

### Disclosure

### Technical Problem

The present disclosure provides a medical liquid injection device that drives safely and can precisely deliver drugs.

### Technical Solution

As a means to achieve the above-described technical problem, a medical liquid injection device according to an embodiment of the present disclosure may comprise: a reservoir assembly storing medical liquid in a storage space defined by a reservoir and a plunger that moves linearly within the reservoir, a needle assembly connected fluidly to the reservoir assembly to discharge the stored chemical liquid, and a driving unit transmitting a driving force generated by a driving module to the plunger, wherein the driving unit may include a rod member coupled to the plunger and moving linearly with the plunger, a wheel member rotating by the driving force, and a connection member having a catching portion that is movable in an axial direction of the wheel member but whose rotation is limited with respect to the wheel member around the axial direction.

In an embodiment, the rod member and the connection member are screw-coupled together, and the connection member is slidably inserted into a through-hole formed in the wheel member, so that it may rotate together with the wheel member.

In an embodiment, a force-applying member inserted in the connection member and disposed between the reservoir assembly and the wheel member may be further included.

In an embodiment, the force-applying member is in close contact with an outer periphery of the connection member and may generate a resisting force in the connection member when the connection member moves.

In an embodiment, the wheel member has a through-hole into which the connection member is inserted, and the through-hole may have a shape corresponding to the catching portion.

Other aspects, features and advantages in addition to those described above will become apparent from the following drawings, claims and a detailed description of the disclosure.

### Advantageous Effects

According to a medical liquid injection device according to an embodiment of the present disclosure, medical liquid may be safely injected into a user. By providing a medical liquid injection device with a simple configuration, the time, cost, and effort required to manufacture the device may be reduced.

The field of the present disclosure is not limited by these effects.

### Description of Drawings

FIG. 1 is a block diagram showing a medical liquid injection system according to an embodiment of the present disclosure.
FIG. 2 is a perspective view showing a medical liquid injection device according to an embodiment of the present disclosure.
FIG. 3 is an exploded perspective view of the medical liquid injection device of FIG. 2.
FIG. 4 is a diagram illustrating a reservoir assembly of FIG. 2.
FIG. 5 is a perspective view showing a driving unit coupled to a plunger of FIG. 2.
FIG. 6 is an exploded perspective view of the driving unit of FIG. 5.
FIG. 7 is a diagram showing a connection member coupled to a wheel member.
FIG. 8 is a diagram showing a state before medical liquid is stored into a reservoir assembly.
FIG. 9 is a diagram showing a state in which medical liquid is stored into a reservoir assembly.

### Best Mode

Since the present disclosure can be modified in various ways and may have various embodiments, specific embodiments will be illustrated in drawings and described in detail in a detailed description. The effects and features of the present disclosure and the methods for achieving thereof will become clear with reference to the embodiments described in detail below along with the drawings. However, the present disclosure is not limited to the embodiments disclosed below and may be implemented in various forms.

Hereinafter, the embodiments of the present disclosure will be described in detail with reference to the accompanying diagrams and when describing with reference to the diagrams, identical or corresponding components will be assigned the same reference numerals, and the redundant description thereof will be omitted.

In the following embodiments, singular terms include plural terms, unless the context clearly dictates otherwise.

In the following embodiments, terms such as include or have mean the presence of features or components described in the specification, and do not exclude in advance the possibility of adding one or more other features or components.

In cases where an embodiment may be implemented differently, a specific process order may be performed differently from the described order. For example, two processes described in succession may be performed substantially at the same time or may be performed in an order opposite to that in which they are described.

In the diagrams, the sizes of components may be exaggerated or reduced for convenience of description. For example, the size and thickness of each component shown in the diagrams are arbitrarily shown for convenience of description, so the following embodiments are not necessarily limited to what is shown.

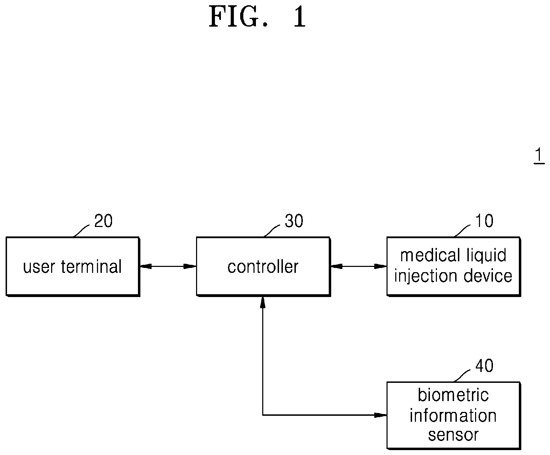

FIG. 1 is a block diagram showing a medical liquid injection system 1 according to an embodiment of the present disclosure.

Referring to FIG. 1, the medical liquid injection system 1 may include a medical liquid injection device 10, a user terminal 20, a controller 30, and a biometric information sensor 40. The medical liquid injection system 1 allows a user to drive and control the system by using the user terminal 20, and medical liquid may be periodically injected from the medical liquid injection device 10 based on glucose information monitored by the biometric information sensor 40.

The medical liquid injection device 10 also performs a function of injecting medical liquid to be injected into a user, such as insulin, glucagon, anesthetics, analgesics, dopamine, growth hormones, and smoking cessation aids, based on data sensed by the biometric information sensor 40.

Additionally, the medical liquid injection device 10 may transmit a status message thereof to the controller 30, including information about the remaining battery capacity of the device, whether the device was successfully booted, whether the injection was successful, or the like. The messages delivered to the controller 30 may be delivered to the user terminal 20 through the controller 30. Alternatively, the controller 30 may transmit improved data processed from the received messages to the user terminal 20.

In an embodiment, the medical liquid injection device 10 is provided separately from the biometric information sensor 40 and may be installed at a distance from a user. In another embodiment, the medical liquid injection device 10 and the biometric information sensor 40 may be provided in one device.

In an embodiment, the medical liquid injection device 10 may be mounted on a user's body. Additionally, in another embodiment, the medical liquid injection device 10 may be mounted on an animal to inject medical liquid.

The user terminal 20 may receive an input signal from a user to drive and control the medical liquid injection system 1. The user terminal 20 may generate a signal that drives the controller 30 and control the controller 30 to drive the medical liquid injection device 10. Additionally, the user terminal 20 may display biometric information measured from the biometric information sensor 40 and status information of the medical liquid injection device 10.

The user terminal 20 refers to a communication terminal that may be used in a wired or wireless communication environment. For example, the user terminal 20 may include a smartphone, a tablet PC, a PC, a smart TV, a mobile phone, a personal digital assistant (PDA), a laptop, a media player, a micro server, a global positioning system (GPS) device, an e-book terminal, a digital broadcasting terminal, navigation, a kiosk, a MP3 player, a digital camera, a home appliance, a camera-equipped device, and other mobile or non-mobile computing devices. Additionally, the user terminal 2 may be a wearable device such as a watch, glasses, a hair band, or a ring equipped with a communication function and data processing function. However, as described above, terminals equipped with applications capable of Internet communication may be borrowed without any limitation.

The user terminal 20 may be connected one-to-one with the pre-registered controller 30. The user terminal 20 may be encrypted and connected to the controller 30 to prevent the controller 30 from being driven and controlled by an external device.

In an embodiment, the user terminal 20 and the controller 30 may be separated and provided as separate devices. For example, the controller 30 may be provided to a user equipped with the medical liquid injection device 10, and the user terminal 20 may be provided to a user or a third party. The user terminal 20 is driven by a guardian, thereby improving the safety of the medical liquid injection system 1.

In another embodiment, the user terminal 20 and the controller 30 may be provided as one device. The controller 30, which is provided as one with the user terminal 20, may communicate with the medical liquid injection device 10 to control an injection of medical liquid.

The controller 30 may perform a function of transmitting and receiving data with the medical liquid injection device 10, transmit a control signal related to the injection of medical liquid such as insulin to the medical liquid injection device 10, and receive a control signal related to measurement of biometric values such as glucose levels from the biometric information sensor 40.

For example, the controller 30 may transmit an instruction request to measure a user's current state to the medical liquid injection device 10 and receive measurement data from the medical liquid injection device 10 in response to the instruction request.

The biometric information sensor 40 may perform a function of measuring a user's biometric values, such as glucose level, blood pressure, and heart rate, depending on the purpose. Data measured by the biometric information sensor 40 may be transmitted to the controller 30, and the cycle and/or injection amount of medical liquid may be set based on the measured data. Data measured by the biometric information sensor 40 may be transmitted to the user terminal 20 and displayed.

For example, the biometric information sensor 40 may be a sensor that measures the user's glucose level. It may be a continuous glucose monitoring (CGM) sensor. A continuous glucose measurement sensor is attached to a user and may continuously monitor glucose levels.

The user terminal 20, the controller 30, and the medical liquid injection device 10 may communicate by using a network. For example, networks include Local Area Network (LAN), Wide Area Network (WAN), Value Added Network (VAN), mobile radio communication network, satellite communication network, and their It is a comprehensive data communication network that includes mutual combinations and allows each network member to communicate smoothly with each other, and may include wired Internet, wireless Internet, and mobile wireless communication networks. Additionally, wireless communications include, for example, wireless LAN (Wi-Fi), Bluetooth, Bluetooth low energy, ZigBee, WFD (Wi-Fi Direct), UWB (ultra wideband), IrDA (infrared Data Association), NFC (Near Field Communication), 5G, or the like, but they are not limited thereto.

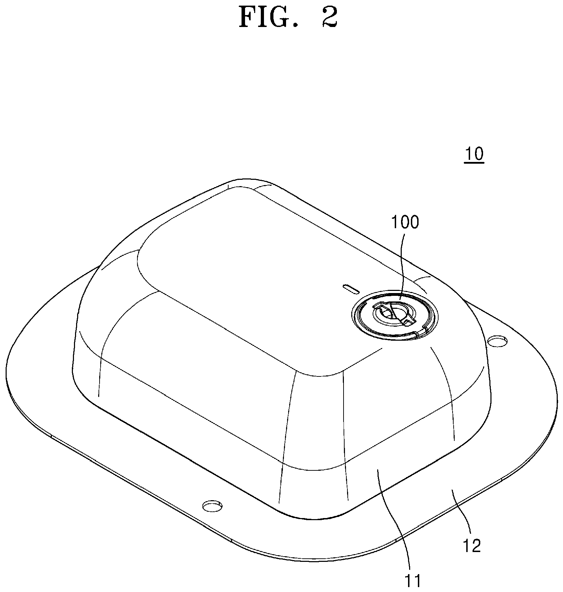

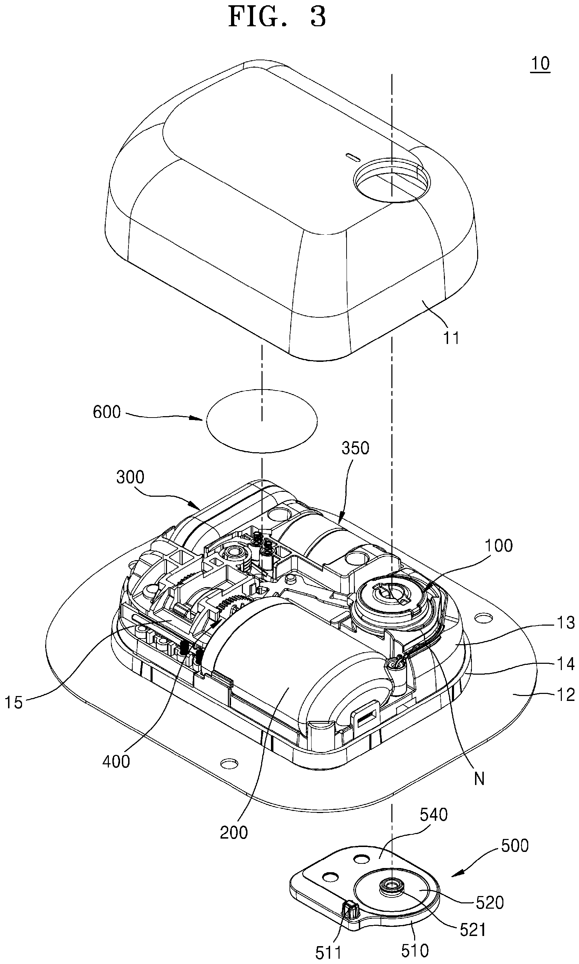

FIG. 2 is a perspective view showing the medical liquid injection device 10 according to an embodiment of the present disclosure, and FIG. 3 is an exploded perspective view of the medical liquid injection device 10 of FIG. 2.

Referring to FIGS. 2 and 3, the medical liquid injection device 10 may inject medical liquid stored inside in a fixed amount set to a user in the state of being attached to the user, the target of injecting medical liquid.

The medical liquid injection device 10 may be used for a variety of purposes depending on the type of medical liquid to be injected. For example, the medical liquid may include medical liquid based on insulin for diabetic patients, medical liquid for pancreas, medical liquid for heart, and other various types of the medical liquid.

An embodiment of the medical liquid injection device 10 may include a housing 11 covering an outer side and an attachment portion 12 located adjacent to a user's skin. The medical liquid injection device 10 includes a plurality of parts disposed in a space between the housing 11 and the attachment portion 12. A separate joining means may be further interposed between the attachment portion 12 and the user's skin, and the medical liquid injection device 10 may be fixed to the skin by the joining means.

The medical liquid injection device 10 may include a needle assembly 100, a reservoir assembly 200, a driving module 300, a battery 350, a driving unit 400, a needle cover assembly 500, and an alarm unit 600.

In the medical liquid injection device 10, at least one of the base bodies may form a frame supporting internal parts. The base body may have a first body 13, a second body 14, and a third body 15 depending on the arrangement.

The first body 13 is disposed below the housing 11, and the needle assembly 100, the reservoir assembly 200, the driving module 300, the battery 350, or the like may be supported in each opening or groove The second body 14 is disposed below the first body 13 and may be connected to the attachment portion 12. The second body 14 may cover the lower portion of the medical liquid injection device 10. The third body 15 is disposed on the upper side of the first body 13 and may support the reservoir assembly 200, the driving module 300, the battery 350, the driving unit 400, or the like in each opening or groove. In the drawing, the first body 13, the second body 14, and the third body 15 are shown, but they are not limited thereto and may be provided as one body or in a plural number.

A control unit (not shown) may be disposed inside the medical liquid injection device 10. The control unit, which is a circuit board, is disposed below the second body 14 and may control the overall driving of the medical liquid injection device 10. The control unit may be in an electrical contact with the driving module 300, the alarm unit 600, and a plurality of sensors (not shown) to control their operation.

When a user rotates the needle assembly 100, the medical liquid injection device 10 may insert a cannula into the user and drive the driving module 300 to start injecting medical liquid.

The driving force generated by the driving module 300 is transmitted to the reservoir assembly 200 by the driving unit 400, so that the medical liquid stored in the reservoir assembly 200 may be discharged. The needle assembly 100 is fluidly connected to the reservoir assembly 200, so that the medical liquid discharged from the reservoir assembly 100 may move to the needle assembly 100. The medical liquid discharged through the needle assembly 100 may be injected into the user through the needle and cannula.

The driving module 300 may be provided with any type of device generating driving force by electricity. For example, all types of pumps, such as mechanical displacement micropumps and electromagnetic motion micropumps may be used. A mechanical displacement micropump is a pump that uses movement of solids or fluids, such as gears or diagrams, to create a pressure difference to induce flow of fluid, and includes a diaphragm displacement pump, a fluid displacement pump, a rotary pump, or the like. An electromagnetic motion micropump is a pump that uses energy in a form of electricity or magnetism directly to movement of fluids, and includes an electrohydrodynamic pump (EHD), an electroosmotic pump, a magnetohydrodynamic pump, an electro wetting pump, or the like.

The battery 350 may supply electricity to the medical liquid injection device 10 to activate each part. In the drawing, a pair of batteries 350 are shown, but they are not limited thereto, and may be set in various ways depending on the capacity, usage range, usage time, or the like of the medical liquid injection device 10. The battery 350 is disposed adjacent to the driving module 300 and may supply electricity to the driving module 300. In addition, the battery 350 is connected to the control module, and may measure data of a rotational number or a rotational speed of the driving unit 400, the amount of medical liquid stored in the reservoir assembly 200, the amount of the injection to a user, based on an electrical signal measured by a sensor unit.

The driving unit 400 is installed between the driving module 300 and the reservoir assembly 200, so that the driving module 300 and the reservoir assembly 200 may be driveably connected to each other to transmit the driving force generated by the driving module 300 to the reservoir assembly 200.

The needle cover assembly 500 may be mounted below the needle assembly 100. The needle cover assembly 500 may prime air stored in the reservoir assembly 200 before injecting medical liquid. When the medical liquid enters the reservoir 210 through a medical liquid injector (NI), gas (air) remaining in the reservoir 210 may be discharged to the outside.

The needle cover assembly 500 may have a first cover 510, a second cover 520, a filter member (not shown), and an adhesive layer (not shown).

The first cover 510 may be disposed below the medical liquid injection device 10. The second cover 520 may be inserted into the opening of the first cover 510 and assembled.

The second cover 520 is assembled to the first cover 510, and a needle and/or cannula may be aligned at the center. The second cover 520 is penetrated in the center in a height direction and may have a storage space in which medical liquid D is stored.

The first cover 510 has greater rigidity than the second cover 520. The first cover 510 is a portion exposed to the outside and is made of a material with somewhat high rigidity. The second cover 520 is assembled to the first cover 510 and is made of a material with less rigidity than the first cover 510 to be inserted into the opening of the third body 15.

The center of the second cover 520 may be provided with a protruding portion 521 inserted into the third body 15. Additionally, the second cover 520 has a fixing protrusion, and the fixing protrusion is inserted into the first cover 510, so that the first cover 510 and the second cover 520 may be assembled.

The protruding portion 521 of the second cover 520 is inserted into the opening at the bottom of the third body 15. The diameter of the protruding portion 521 is set to be slightly larger than the diameter of the opening. Since the second cover 520 has a certain amount of elasticity, the protruding portion 521 may be inserted into the opening and fixed thereto.

The second cover 520 has an inner diameter, so that the needle and cannula may be aligned on the top. The inner diameter of the second cover 520 forms a storage space of the second cover 520, where medical liquid may be stored or gas may be moved and discharged.

The filter member is mounted on the second cover 520. The filter member is disposed below the storage space of the second cover 520, and gas such as air pass through the filter member, but liquids such as medical liquid do not pass therethrough. Thus, the air discharged from the needle passes through the filter member and is discharged to the outside, but the medical liquid discharged from the needle may be stored in a storage space defined by the second cover 520 and the filter member.

A shape of the filter member may change depending on the amount of medical liquid stored in the storage space. For example, when the storage space is filled with medical liquid, the filter member expands downward, allowing a user to recognize that the medical liquid has entered the needle cover assembly 500.

The adhesive layer is disposed on one side of the needle cover assembly 500, and the needle cover assembly 500 may be attached to the attachment portion 12.

The alarm unit 600 is disposed inside or outside the medical liquid injection device 10 and may notify the user of a normal operation or a malfunction of the medical liquid injection device 10.

For example, the alarm unit 600 is disposed below the housing 11 and connected to a circuit board. The alarm unit 600 may generate a warning sound or light to deliver an alarm to an external user.

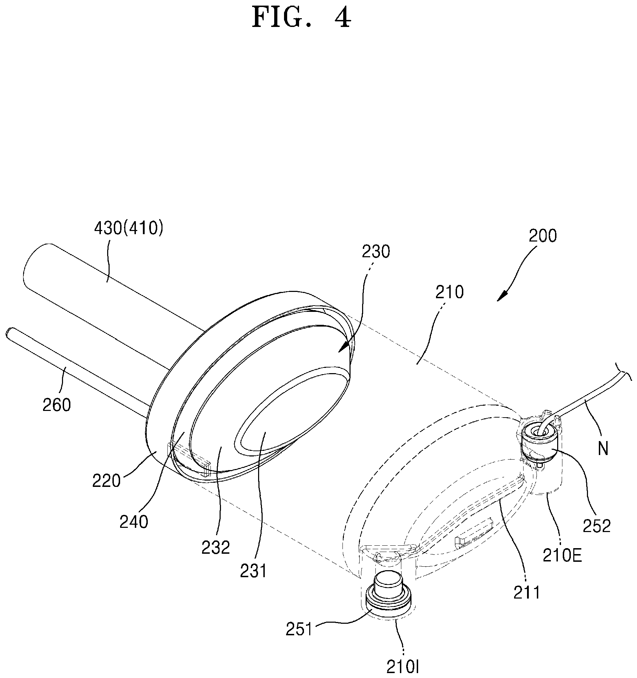

FIG. 4 is a diagram illustrating the reservoir assembly 200 of FIG. 2.

Referring to FIG. 4, the reservoir assembly 200 may include a reservoir 210, a cap cover 220, a plunger 230, a sealing ring 240, and a sensing unit 260.

The reservoir 210 forms an exterior of the reservoir assembly 200 and may provide an internal space in which the plunger 230 may be accommodated.

In an embodiment, the reservoir 210 may be formed in the shape of a cylinder extending in a forward and backward direction. A cross-section of the reservoir 210 may be formed in an oval to prevent the plunger 230 from rotating in the internal space of the reservoir 210. Since the cap cover 220 is mounted at the rear of the reservoir 210, an internal space separated from the outside may be defined by the reservoir 210 and the cap cover 220, and a connector member 233 or a rod member 410 and a connection member 460 may move through an opening (not shown) disposed in the cap cover 220.

The plunger 230 is accommodated in the internal space and may move linearly in a forward and backward direction. In an embodiment, the plunger 230 may move from front to back by medical liquid flowing into an internal space. Additionally, it may move from back to front by a driving force generated by a driving module. As the plunger 230 advances, the medical liquid stored in the reservoir 210 may flow into a needle assembly.

A portion of the internal space may be defined as a storage space where medical liquid is stored by the reservoir 210 and the plunger 230. A volume of the storage space may be changed by the plunger 230 moving in the internal space. For example, if the plunger 230 is disposed at the forefront of the internal space and an end 231 and an inclined surface 232 of the plunger 230 are in close contact with the reservoir 210, a storage space may not be formed in the internal space, but as medical liquid flows into the reservoir 210, a storage space is formed, and the volume of the storage space may be increased by the medical liquid flowing into the reservoir 210. Additionally, as the stored medical liquid is discharged from the reservoir 210, the volume of the storage space may decrease.

The plunger 230 may be coupled with the connector member 233 extending forward and backward. The connector member 233 is installed on the plunger 230, moves together with a movement of the plunger 230, and may sense the amount of stored medical liquid.

The plunger 230 is provided with a sealing ring 240 at a portion in contact with an inner wall of the reservoir 210, so that when the plunger 230 moves, medical liquid may be prevented from leaking through a space between the plunger 230 and the inner wall of the reservoir 210.

A plurality of openings may be formed in front of the reservoir 210 to communicate the internal space with the outside. The plurality of openings may be an inlet 2101 and an outlet 210E. In an embodiment, the inlet 2101 and the outlet 210E may each be formed as openings penetrating the front of the reservoir 210. In another embodiment, the inlet 210I and the outlet 210E may communicate with the interior of the reservoir 210 through a flow path 211 formed as a groove on a front inner surface of the reservoir 210.

The inlet 210I may provide a flow path through which external medical liquid flows into the internal space of the reservoir 210. A first packing member 251 is disposed at the inlet 210I to pack the inlet 210I and may prevent medical liquid from leaking through the inlet 210I. The first packing member 251 may prevent medical liquid from leaking and inject the medical liquid while an injection needle (not shown) penetrates the first packing member 251.

The first packing member 251 may have resealability. For example, even if the injection needle is removed after penetrating the first packing member 251 to fill the reservoir 210 with medical liquid, the first packing member 251 may prevent the medical liquid from leaking through the portion where the injection needle has penetrated. The first packing member 251 may be formed of a material containing, for example, polypropylene, thermoplastic elastomer, vegetable oil, or the like. The medical liquid injected by the injection needle pushes the plunger 230 backward and flows into the internal space of the reservoir 210 to form a storage space.

A conduit (N) may be connected to the outlet (210E), and the reservoir 210 may be connected to the needle assembly through the conduit (N). A second packing member 252 may be provided between the outlet 210E and the conduit N to prevent medical liquid from leaking between the outlet 210E and the conduit N. The medical liquid stored in the reservoir 210 may flow to the needle assembly through the outlet 210E and the conduit.

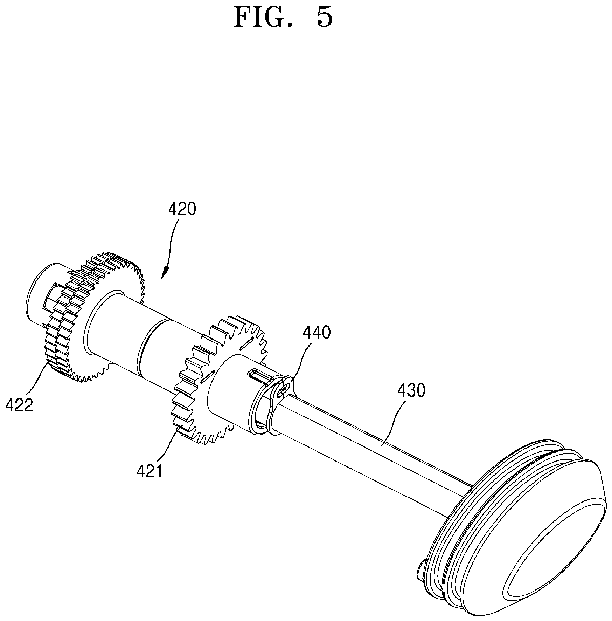

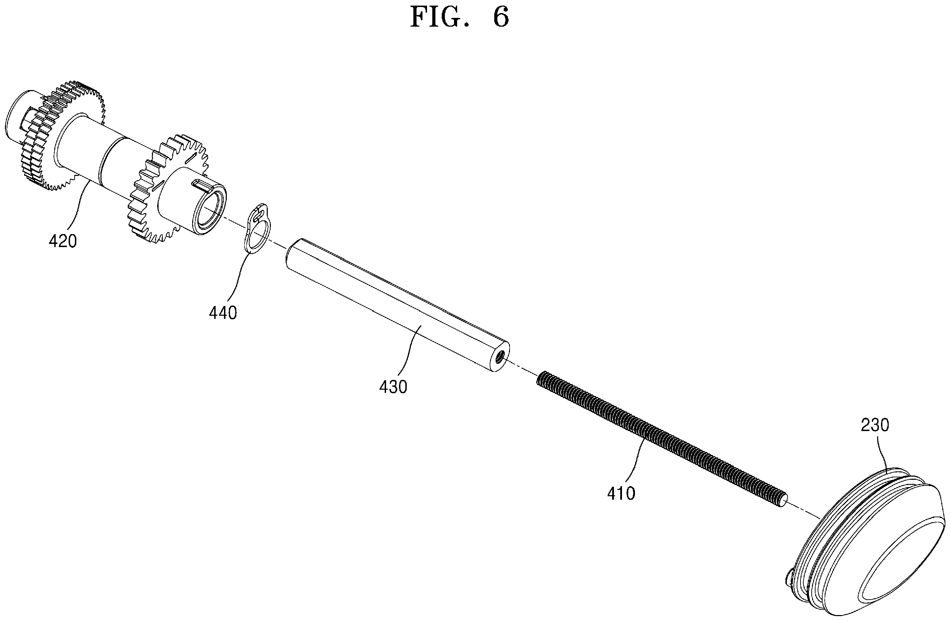

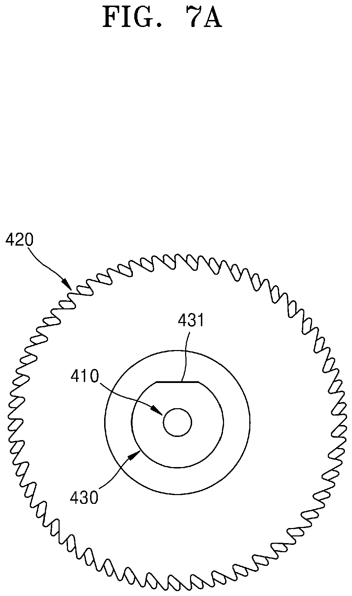

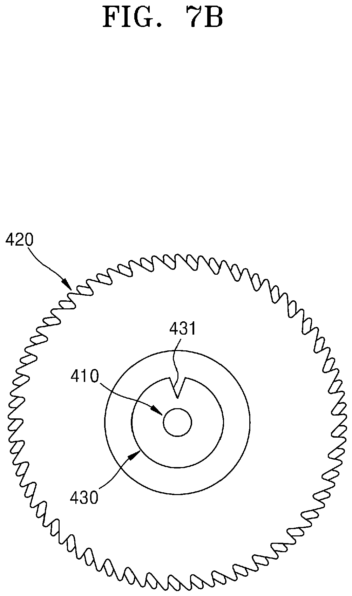

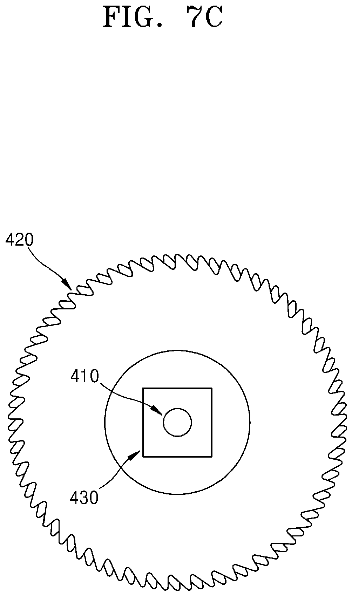

FIG. 5 is a diagram showing the driving unit 400 of FIG. 2, FIG. 6 is an exploded perspective view of the driving unit 400 of FIG. 5, and FIG. 7 is a diagram showing a connection member coupled to a wheel member.

A driving unit may driveably connect a driving module and a reservoir assembly. Referring to FIGS. 5 and 6, the driving unit may include a rod member 410, a wheel member 420, a connection member 430, and a force-applying member 440. The driving unit may transmit the driving force generated by the driving module to the reservoir assembly, preferably to the plunger 230.

The rod member 410 may be formed extending in one direction. An extending direction of the rod member 410 may coincide with an extending direction of the reservoir. One side of the rod member 410 is coupled to the plunger 230 and may move linearly in one direction along with the movement of the plunger 230. As the plunger 230 moves, a portion of the rod member 410 may be variably accommodated in the internal space. The other side of the rod member 410 may be disposed outside the reservoir 210.

The connection member 430 may be formed extending in one direction together with the rod member 410. The connection member 430 may be coupled to the rod member 410 and driveably connected to the plunger 230. The driving force transmitted to the connection member 430 may be transmitted to the plunger 230 through the rod member 410.

The connection member 430 may be coupled to portions which are coupled to and opposite the plunger 230 of the rod member 410. The connection member 430 is able to move linearly together with the rod member 410 along a linear movement of the plunger 230.

The connection member 430 and the rod member 410 may be coupled so that the rod member 410 is linearly movable when the connection member 430 rotates. In an embodiment, the rod member 410 and the connection member 430 may be screw-coupled together. For example, the rod member 410 may have a through-hole inside extending in one direction, and screw threads formed on an inner peripheral surface of the rod member 410 may be screw-coupled together with screw threads formed on an outer peripheral surface of the connection member 430. Alternatively, the connection member 430 may have a through-hole inside formed extending in one direction, and the screw threads formed on the inner peripheral surface of the connection member 430 may be screw-coupled together with the screw threads formed on the outer peripheral surface of the rod member 410.

The wheel member 420 is driveably connected to the driving module and may rotate by the driving force generated by the driving module. The wheel member 420 has a first wing portion 421 and a second wing portion 422 and may be provided with a through-hole space in which the connection member 430 may move. The through-hole may be formed inside the wheel member 420 extending in one direction.

Since at least one of the first wing portion 421 and the second wing portion 422 is always driveably connected to the driving module, the wheel member 420 may rotate when the driving module is driven. For example, the first wing portion 421 and the second wing portion 422 may have a gear tooth shape on outer peripheral surfaces thereof. A connector (not shown) connected to the driving module may apply the gear teeth to rotate the wheel member 420.

The connection member 430 may move along the through-hole formed in the wheel member 420 and may be coupled to the wheel member 420 to rotate with the rotation of the wheel member 420. In an embodiment, the through-hole formed in the wheel member 420 may be formed in a shape corresponding to the connection member 430 so that the connection member 430 is inserted into the wheel member 420 to slide. At this time, a catching portion may be provided on one side of the connection member 430.

The catching portion may form a load at a portion where an outer side of the connection member 430 and an inner side of the wheel member 420 meet each other.

In an embodiment, referring to FIG. 7A, a catching portion 431 formed on the connection member 430 whose outer side is cylindrical may be provided with a cut surface 431 formed by plane cutting one side of the connection member 430 along an extending direction of the connection member 430. Accordingly, when the wheel member 420 rotates, a load is generated between the cut surface 431 formed on the connection member 430 and the inner surface of the wheel member 420 in contact therewith, so the connection member 430 may rotate together with the wheel member 420.

In another embodiment, referring to FIG. 7B, the catching portion 431 may be provided with a notch portion 431 formed on one side of the connection member 430 whose outer side is cylindrical along an extending direction of the connection member 430. A portion of the inner side of the wheel member 420 may be inserted into the notch portion 431 in a corresponding shape, so that when the wheel member 420 rotates, the connection member 430 may rotate together with the wheel member 420 by a load generated between the notch portion 431 and the inner surface of the wheel member 420 in contact therewith.

In FIG. 7B, an embodiment is shown in which the notch portion 431 is formed on the outer side of the connection member 430, but it is not limited thereto, and the notch portion may be formed on the inner surface of the wheel member 420 and a portion of the outer side of the connection member 430 in a corresponding shape may be inserted.

At least one notch portion may be formed, and a plurality of notch portions may be arranged around an axis.

In another embodiment, referring to FIG. 7C, a cross-section of the connection member 430 may be formed into a square. At this time, the outer surface of the connection member 430 corresponding to a corner of the square may act as a catching portion. Accordingly, when the wheel member 420 rotates, a load is generated between each outer surface of the connection member 430 and the inner surface of the wheel member 420 in contact therewith, and the connection member 430 may rotate together with the wheel member 420.

In FIG. 7C, an embodiment is shown in which a cross-section of the outer side of the connection member 430 is formed as a square, but it is not limited thereto, and the cross-section of the outer side of the connection member 430 may be formed in a polygon such as a triangle, a pentagon, or the like.

With the connection member 430 inserted into the through-hole formed in the wheel member 420, when the wheel member 420 rotates, a load due to the rotation of the wheel member 420 is applied on a catching portion, so that the connection member 430 may rotate with the rotation of the wheel member 420. That is, the catching portion limits the rotation of the connection member 430 with respect to the wheel member 420, so that the connection member 430 may rotate together with the wheel member 420.

The connection member 430 is disposed between the rod member 410 and the wheel member 420, so that a driving force transmitted to the wheel member 420 may be transmitted to the rod member 410. Regardless of the driving force generated by the driving module, the connection member 430 and the rod member 410 are linearly movable together. In addition, when the driving force generated by the driving module is transmitted to the wheel member 420, the connection member 430 rotates together with the wheel member 420 and may linearly move only the rod member 410. In an embodiment, the connection member 430 may be slidably inserted into a through-hole formed in the wheel member 420 while being screw-coupled together with the rod member 410.

The force-applying member 440 may apply a force to the connection member 430 opposite to the direction in which the connection member 430 moves. In an embodiment, the force-applying member 440 may rub against an outer peripheral surface of the connection member 430, thereby restricting the linear movement of the connection member 430. For example, the force-applying member 440 may be formed in a ring shape in close contact with the outer periphery of the connection member 430. Alternatively, the force-applying member 440 may be formed in a clip-shaped elastic body to apply a force to the outer side of the connection member 430. The force-applying member 440 may generate a frictional force according to the movement of the connection member 430, while being fixed with respect to the linear movement of the connection member 430. In another embodiment, the force-applying member 440 is formed of an elastic body such as a spring and may apply a force to the connection member 430 in a direction opposite to the direction in which the connection member 430 moves.

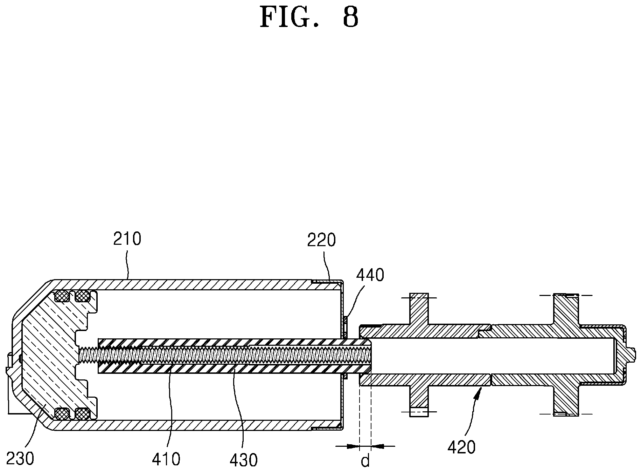

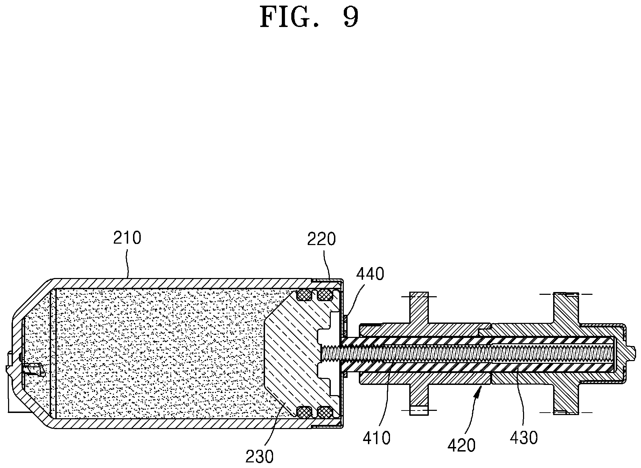

FIG. 8 is a diagram showing a state before medical liquid is stored in a reservoir assembly, and FIG. 9 is a diagram showing a state in which medical liquid D is stored in a reservoir assembly.

Referring to FIGS. 8 and 9, the mechanism by which the medical liquid D is stored in the reservoir assembly and the medical liquid D stored in the reservoir assembly is discharged will be described as follows.

Referring to FIG. 8, before the medical liquid is injected into the reservoir assembly, the plunger 230 may be disposed in front of an internal space of the reservoir 210. A portion of the rod member 410 coupled to a rear end of the plunger 230 along with the connection member 430 coupled thereto may extend backward through an opening formed in a cap cover.

The rear end of the connection member 430 may be inserted into a through-hole formed in the wheel member 420 to a predetermined depth d. A portion of the connection member 430 is inserted into the through-hole formed in the wheel member 420 so that when the connection member 430 slides into the through-hole formed in the wheel member 420, it is possible to prevent the connection member 430 and the wheel member 420 from colliding at the entrance of the through-hole formed in the wheel member 420.

A force-applying member 440 may be disposed on one side of the connection member 430. The force-applying member 440 may be fixed in close contact with an outer peripheral surface of the connection member 430. The force-applying member 440 in close contact with the outer peripheral surface of the connection member 430 may not generate a frictional force when no external force act on the connection member 430. When an external force is applied on the connection member 430, the force-applying member 440 may apply a frictional force opposing the external force to the connection member 430 in response. The movement of the connection member 430 may be restricted by the force applied by the force-applying member 440.

Before an injection of medical liquid into the reservoir 210 is completed, the plunger 230 may move backward, so when an external factor such as an impact is applied on the device, the plunger 230 moves backward and gas may flow into the reservoir 210. By restricting the connection member 430 to move, the force-applying member 440 may prevent gas from flowing into the reservoir 210, before an injection of medical liquid into the reservoir 210 is completed.

The rod member 410 and the connection member 430 may be movable together and may be coupled to be movable relative to each other. For example, the rod member 410 and the connection member 430 may be screw-coupled together by screw threads formed on the outer peripheral surface of the rod member 410 and screw threads formed on the inner peripheral surface of the connection member 430. Accordingly, the rod member 410 and the connection member 430 move simultaneously, and when the connection member 430 rotates, the rod member 410 may move relative to the connection member 430.

Medical liquid may flow into the reservoir 210 through an inlet formed in front of the reservoir 210 by a separately provided medical liquid injector. The medical liquid flows between the reservoir 210 and the plunger 230, and the plunger 230 may be pushed backward to form a storage space. Additionally, a volume of the storage space may be gradually increased and the plunger 230 may be moved backward. The rod member 410 and the connection member 430 connected to the plunger 230 move backward with the movement of the plunger 230 and may be inserted while sliding into a through-hole formed in the wheel member 420.

When the connection member 430 moves backward due to the medical liquid flowing into the reservoir 210, the force-applying member 440, which is fixed in close contact with the outer peripheral surface of the connection member 430, may generate a frictional force on the outer peripheral surface of the connection member 430 and may apply a force opposite to the direction in which the connection member 430 moves. Accordingly, a positive pressure corresponding to the force formed by the force-applying member 440 may be additionally formed inside the storage space.

As shown in FIG. 8, when the injection of the medical liquid D into the reservoir 210 is completed, the plunger 230 may be disposed at the rear of the internal space of the reservoir 210.

A positive pressure may be formed inside the storage space as medical liquid is injected, but when the injection of the medical liquid is completed, due to a frictional force between the plunger 230 and the reservoir 210 or a frictional force caused by the force-applying member 440, or due to a cap member provided at the rear of the reservoir 210, the plunger 230 may be disposed at the rear of the internal space without moving any further backwards.

As the driving module is driven, an injection of medical liquid may be started. The driving force generated by the driving module is transmitted to the plunger 230 through the driving unit, so that the medical liquid stored in the reservoir 210 may be discharged into the needle assembly.

When the driving force generated by the driving module is transmitted to the wheel member 420, the wheel member 420 may rotate. While the connection member 430 coupled to the wheel member 420 rotates together with the wheel member 420, the rod member 410 coupled to the plunger 230 does not rotate and may move forward by being screw-coupled together with the connection member 430. At this time, if a screw coupling is formed only on a portion of the outer peripheral surface of the rod member 410 and a portion of the inner peripheral surface of the connection member 430, when the wheel member 420 and the connection member 430 rotate and the rod member 410 moves forward, the load due to the screw coupling between the connection member 430 and the rod member 410 may be reduced. Accordingly, screw threads which are coupled to screw threads formed on the outer peripheral surface of the rod member 410 may be formed only on a portion of the inner peripheral surface of the connection member 430.

When the rod member 410 moves forward, the plunger 230 moves forward together with the rod member 410 and discharges the medical liquid D stored in the storage space into the needle assembly.

The force-applying member 440 may facilitate discharging medical liquid from the needle assembly. A predetermined pressure is required to inject medical liquid through a needle and a cannula inserted into a user, but an injection of medical liquid may be facilitated due to the positive pressure created in the storage space by the force-applying member 440. When the driving force generated by the driving module is transmitted to the plunger 230, the plunger 230 responds quickly to the transmitted driving force and may move smoothly.

As such, the present disclosure has been described with reference to an embodiment shown in the drawings, but this is merely an example, and a person with ordinary skill in the art will understand that various modifications and variations of the embodiments are possible therefrom. Therefore, the field of the true technical protection of the present disclosure should be determined by the technical spirit of the attached claims.

### Industrial Applicability

According to the present disclosure, a medical liquid injection device is provided. In addition, embodiments of the present disclosure can be applied to devices for industrial use, such as those for injecting a medical liquid into a patient's body.

## Claims

1. A medical liquid injection device, comprising:
a reservoir assembly storing medical liquid in a storage space defined by a reservoir and a plunger moving linearly within the reservoir,
a needle assembly fluidly connected to the reservoir assembly to discharge stored medical liquid, and
a driving unit configured to transmit a driving force generated by a driving module to the plunger,
wherein the driving unit comprises
a rod member coupled to the plunger and moving linearly with the plunger, a wheel member rotating by the driving force, and
a connection member which is movable in an axial direction of the wheel member and has a catching portion whose rotation with respect to the wheel member is limited around the axial direction.

2. The medical liquid injection device of claim 1,
wherein the rod member and the connection member are screw-coupled together,
wherein the connection member is slidably inserted into a through-hole formed in the wheel member and rotates together with the wheel member.

3. The medical liquid injection device of claim 1, further comprising
a force-applying member fit around the connection member and disposed between the reservoir assembly and the wheel member.

4. The medical liquid injection device of claim 3,
wherein the force-applying member is in close contact with an outer periphery of the connection member and generates a resisting force in the connection member when the connection member moves.

5. The medical liquid injection device of claim 1,
wherein the wheel member has
a through-hole into which the connection member is inserted, and the through-hole has a shape corresponding to the catching portion.
